# EUROPÄISCHE PATENTSCHRIFT

(11) **EP 2 169 965 B1**
(45) Veröffentlichungstag und Bekanntmachung des Hinweises auf die Patenterteilung: **21.09.2011**
(21) Anmeldenummer: 08017201.8
(22) Anmeldetag: 30.09.2008
(51) Int. Cl.: H04N 9/73, F21V 23/04, F21V 33/00, G02B 27/28, H05B 37/02, F21Y 101/02

(54) **System mit einer Operationsleuchte, einer Kamera und einem Monitor**
System with an operating light, a camera and a monitor
Système doté d'un éclairage d'opération, d'une caméra et d'un moniteur

(43) Veröffentlichungstag der Anmeldung: 31.03.2010
(73) Patentinhaber: TRUMPF Medizin Systeme GmbH + Co. KG, 07318 Saalfeld (DE)
(72) Erfinder: Marka, Rudolf, Dr., 85737 Ismaning (DE); Fritze, Dirk, 82275 Emmering (DE)
(74) Vertreter: Prüfer & Partner GbR European Patent Attorneys

(56) Entgegenhaltungen:
- EP-A- 0 933 973
- EP-A- 1 568 938
- DE-A1- 19 712 434
- DE-U1-202007 007 054
- JP-A- 2005 198 750

## Beschreibung

Die Erfindung betrifft ein System aus einer Operationsleuchte, deren Farbtemperatur mit Hilfe einer Steuereinrichtung einstellbar ist, einer Kamera und einem Monitor.

Bei Operationsleuchten in LED-Technologie kann zur besseren Unterscheidbarkeit von verschiedenen Gewebetypen die Farbtemperatur des abgegebenen Lichts in einem festgelegten Bereich eingestellt werden. Dadurch ist es möglich, verschiedene Gewebearten besser zu unterscheiden.

Eine Operationsleuchte in LED-Technologie ist beispielsweise aus der EP-A-1722157 bekannt. Hier wird die Farbtemperatur des abgestrahlten Lichts durch unterschiedliche Gewichtung von verschiedene Spektren abstrahlenden Lichtquellen erzeugt. Eine Veränderung der Farbtemperatur ist im Bereich von 3.500 K bis 5.000 K möglich. Die Information, welche Farbtemperatur abgestrahlt wird, ist also in der Steuerung der Operationsleuchte vorhanden. Bei der Veränderung der Farbtemperatur bleibt die Leuchtintensität der Leuchte annähernd konstant. Die Leuchtmittel der Operationsleuchte und deren Ansteuereinheiten sind so kalibriert, dass die Sollvorgaben der Steuerung bezüglich der Farbtemperatur genau in die Istwerte umgesetzt werden.

In einer elektronischen Kamera wird das einfallende Licht durch Sensoren (CCD oder CMOS) in Spannungssignale umgewandelt. Zur Erfassung von farbigen Bildern wird das Licht farbspezifisch von verschiedenen Sensoren aufgefangen, was dann jeweils ein farbspezifisches Spannungssignal ergibt. Bei der Anzeige eines elektronisch aufgenommenen Bildes oder einer Videosequenz ist es erforderlich, einen Weißabgleich durchführen, um das angezeigte Bild oder die angezeigte Videosequenz in realistischen Farben darzustellen.

Je nach Einstellung der Farbtemperatur der Operationsleuchte würde, ohne Weißabgleich, ein bläuliches bis rötliches Bild entstehen. Ein automatischer Weißabgleich, wie bei der Anwendung von konventionellen elektronischen Kameras, ist bei dem Einsatz in diesem Anwendungsgebiet nicht möglich, daher wird die Information über die eingestellte Farbtemperatur von der Operationsleuchte an die Kamera übergeben.

Ein System nach dem Oberbegriff des Anspruchs 1 ist aus der DE-A-20 2007 007 054 bekannt. Dabei erfolgt eine Kommunikation zwischen einem Leuchtenkörper und einer Kamera mittels Kalibrierwerten, welche mittels eines Bussystems von dem Leuchtenkörper an die Kamera übermittelt werden. Die Kalibrierwerte sind experimentell bestimmt. Durch diese Datenübertragung kann die Kamera ihre Weißabgleichparameter entsprechend einstellen, wodurch eine farbgetreue Wiedergabe des abgebildeten OP-Bereiches gewährleistet ist. Die Bildsignale werden an eine Steuereinrichtung der Kamera weitergegeben und an den Signalausgängen der Steuereinrichtung können die Bildsignale für Bildverarbeitungsgeräte oder einen Monitor abgenommen werden. Mit der Einstellung der Farbtemperatur am Leuchtenkörper erfolgt eine Anpassung der Kamera während der Übertragung der Bildsignale an das Bildverarbeitungsgerät oder den Monitor, so dass ein Bild mit farbgetreuer Widergabe entsteht.

Hierbei ist es jedoch erforderlich, eine Kamera zu verwenden, die eine solche Einstellmöglichkeit aufweist.

Die Offenlegungsschrift DE-A-197 12 434 offenbart ein Verfahren zum Abgleich eines Videosignals mit einer Bildverarbeitungsvorrichtung mit einer Standard-Videokamera, und einer Abgleichseinheit, die aus einer Weißabgleichseinheit und einer Korrektureinheit besteht. Die Abgleichseinheit gleicht das von der Videokamera gelieferte Videosignal derart ab, dass der Farbeindruck eines durch einen Monitor angezeigten Bildes, unter Berücksichtigung der zur Beleuchtung verwendeten Farbtemperatur, weitestgehend dem Farbeindruck, wie ihn das menschliche Auge wahrnimmt, entspricht. Das abgeglichene Signal wird dann über eine Leitung an einen Moitor übergeben und von diesem angezeigt.

In der Zusammenfassung der japanischen Patentanmeldung JP 2005 198 750 ist eine Anlage für ein elektronisches Endoskopsystem gezeigt, die es einem Bediener ermöglicht, mit einem Einstellschalter einen genauen Farbabgleich einzustellen. Dabei wird eine Bildbearbeitungseinheit durch ein Steuerungsmittel so angesteuert, dass die Bilder durch den Farbabgleich entsprechend der Bedienung des Bedienmittels bearbeitet werden, und das ausgestrahlte Licht entsprechend dem Farbabgleich gesteuert wird.

Der Erfindung liegt die Aufgabe zugrunde, bei der Verwendung einer beliebigen Kamera, einen automatischen Weißabgleich des Bildverarbeitungsgeräts durchführen zu können.

Die Aufgabe wird mit den Merkmalen des Anspruchs 1 gelöst. Weiterbildungen der Erfindung sind in den Unteransprüchen beschrieben.

Ein System der eingangs genannten Art ist so ausgebildet, dass Mittel zur Übertragung der eingestellten Farbtemperatur an den Monitor vorgesehen sind. Die Information über die gewählte Farbtemperatur der Operationsleuchte wird an den Monitor übertragen, welcher seine Weißabgleichparameter entsprechend aufrufen kann.

Weitere Merkmale und Zweckmäßigkeiten der Erfindung werden anhand eines Ausführungsbeispiels unter Bezugnahme auf die beigefügten Zeichnungen erläutert.

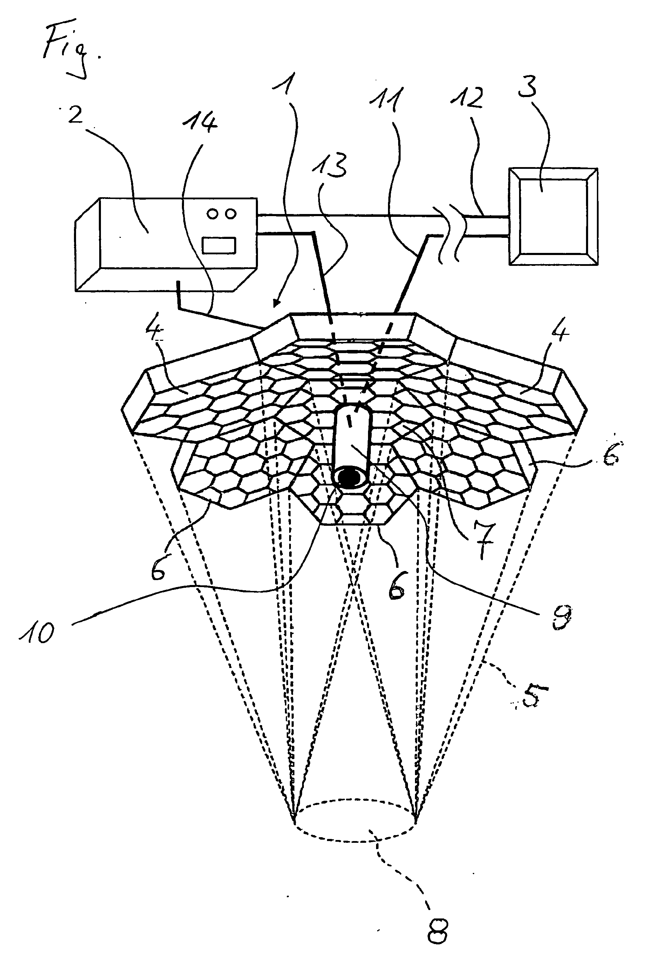

Die Figur zeigt eine schematische Darstellung eines Systems aus einer Operationsleuchte 1, einer Steuereinrichtung 2 und einem Bildverarbeitungsgerät, hier einem Monitor 3.

Die Operationsleuchte 1 ist mit einem nicht gezeigten Tragsystem an einer Raumdecke, einer Wand oder einem fahrbaren Ständer befestigt, so dass innerhalb des Aktionsbereichs jede Position und Orientierung der Operationsleuchte 1 ermöglicht ist, um die Operationsstelle bei einem chirurgischen Eingriff optimal zu beleuchten. An einem Ausleger des Tragsystems ist der Monitor 3 befestigt. Der Monitor 3 kann aber auch auf einer Medizinischen Versorgungseinheit, einem Stativ oder einem Gerätewagen angebracht sein.

Die Operationsleuchte 1 weist Leuchtmittel 4 auf, die durch LEDs mit einem Refraktor ausgebildet sind, welche durch die Bildung von Lichtstrahlen 5 ein Leuchtfeld 8 auf der Operationsstelle ausleuchten. Jedes Leuchtmittel 4 ist so ausgebildet, dass es das gesamte Leuchtfeld 8 ausleuchtet. Es werden verschiedenfarbige Leuchtmittel 4 (warmweiß, kaltweiß, cyan, blau) in einer bestimmten Anzahl verwendet, um das Spektrum des natürlichen Tageslichts zu erzeugen. Durch eine Verstellung der Leistungsdaten der einzelnen Leuchtmittel 4 wird die Farbtemperatur des abgestrahlten Lichts zwischen 3.500 K und 5.000 K verändert.

Die Leuchtmittel 4 sind in sechs äußeren Modulen 6 und in einem inneren Modul 7 angeordnet, wobei die Unterseite der Module 6, 7, die mit Ausnahme eines kleinen Randbereichs die Lichtaustrittsfläche bildet, auf der gesamten Lichtaustrittsfläche mit den Leuchtmitteln 4 ausgefüllt ist. Die äußeren Module 6 sind zu dem inneren Modul 7 verschwenkbar befestigt, um die Lichtstrahlen 5 in verschiedenen Abständen zur Operationsleuchte 1 auf das Leuchtfeld 8 auf der Operationsstelle zu fokussieren

Im Zentrum des inneren Moduls 7 ist ein Griff 9 angeordnet, um die sterile Positionierung der Operationsleuchte 1 zu ermöglichen. Der Griff 9 wird durch eine Hülse aus sterilisierbaren Kunststoff gebildet, der verdrehbar auf einer Griffaufnahme mit Hilfe eines Rastknopfs befestigt ist. Durch Verdrehen des Griffs 9 um einen Winkel von ca. 20° in die verschiedenen Drehrichtungen wird ein jeweils ein Schalter betätigt und über einen Antriebsmotor die äußeren Module 6 entweder nach oben oder nach unten verkippt, um die äußeren Module 6 auf das Leuchtfeld 8 zu fokussieren. Die Hülse des Griffs 9 ist auf der Unterseite mit einer klaren Glasscheibe versehen, um die Durchsicht einer sich innerhalb der Hülse des Griffs 9 befindlichen Kamera 10 zu ermöglichen.

Die Kamera 10 innerhalb der Griffhülse 9 ist als eine CCD-Kamera ausgeführt. Sie ist über einen Bajonettverschluss am Gehäuse der Operationsleuchte 1 befestigt. Durch die Befestigung am Gehäuse der Operationsleuchte 1 wird automatisch eine nicht gezeigte Steckverbindung von einer Versorgungs- und Signalleitung 13 und einer Videoleitung 11 verbunden.

Das aufgenommene Bild wird durch CCD-Sensoren der Kamera 10 in elektrische Signale umgewandelt, die dann entsprechend verstärkt, von einem Signalausgang über die Steckverbindung und die Videoleitung 11 durch das Gehäuse der Operationsleuchte 1 und das Tragsystem zu dem Bildverarbeitungsgerät, in diesem Fall dem Monitor 3 geleitet werden.

In der Kamera 10 sind Motoren vorgesehen, die die Funktionen "Zoom", "Fokussieren" und "Bildaufrichten" ermöglichen. Die "Zoom"-Funktion ermöglicht die Auswahl des Bildausschnitts in einem bestimmten Vergrößerungs- bzw. Verkleinerungsbereich und die "Fokussieren"-Funktion das Scharfstellen des aufgenommenen Bilds. Die "Bildaufrichten"-Funktion ermöglicht es, nach einem Verdrehen der Operationsleuchte 1, das Bild auf dem Monitor 3 so zu orientieren, dass es wieder die Standard-Ausrichtung (z.B. in Blickrichtung des Operateurs) hat.

Die Steuereinrichtung 2 hat die Aufgabe, die Beleuchtungsfunktion der Operationsleuchte 1 und die Funktionen der Kamera 10 zu steuern. Dazu ist die Steuereinrichtung 2 mit der Kamera 10 über die Versorgungs- und Signalleitung 13 und mit der Operationsleuchte 1 über eine Steuerleitung 14 verbunden.

An der Steuereinrichtung 2 sind Bedienelemente zum Ein- und Ausschalten der Operationsleuchte 1, zum Dimmen, also zum Einstellen der Helligkeit, zur Einstellung der Farbtemperatur, zum Einstellen des Zoom-Faktors der Kamera 10 und zur Ansteuerung des Motors zum "Bildaufrichten" vorhanden. Innerhalb der Steuereinrichtung 2 ist eine nicht gezeigte elektronische Steuerung vorgesehen. Die Bedienelemente sind mit der elektronischen Steuerung verbunden und geben Signale an die elektronische Steuerung, die dann die entsprechenden Befehle für die Aktoren, die Motoren der Kamera 10 und die Leistungssteuerungselemente der Leuchtmittel 4 der Operationsleuchte 1 bildet.

Die Steuereinrichtung 2 ist hier als eine separate Baugruppe dargestellt. Es kann aber auch in die Operationsleuchte 1 integriert sein, um die Leitungslängen zu verkürzen.

Die Steuereinrichtung 2 ist mit der Bildverarbeitungseinrichtung 3 über eine Signalübertragungsstrecke, hier einer Signalleitung 12, verbunden und gibt die Signale über eine Schnittstelle (hier: RS 232) an den Monitor 3 weiter.

Der Monitor 3 enthält neben der üblichen Anzeigevorrichtung, einem LCD-Display, und einer Steuerung zur Aufbereitung der Bildsignale von der Kamera 10 zu einem sichtbaren Bild auf dem LCD-Display, einen Speicherbereich zum Abspeichern der Parameterdatensätze (Setup) für die Farbtemperaturkorrektur, dem Weißabgleich, des angezeigten Bildes.

Die Parameter für die Farbtemperaturkorrektur bei den einzelnen, an der Operationsleuchte 1 einstellbaren Farbtemperaturen werden empirisch ermittelt und in dem Speicherbereich abgespeichert. Die Parameter beschreiben die Abweichung des Farborts des übertragenen Videosignals vom tatsächlichen Farbort eines aufgenommenen Bildpunkts.

Weiterhin enthält der Monitor 3 eine Schnittstelle (RS 232) um das Signal der an der Steuereinrichtung 2 eingestellten Farbtemperatur, das von der Steuereinrichtung 2 über die Signalleitung 12 empfangen wird, wieder in einen von der Steuerung des Monitors 3 erkennbaren Datensatz umzuwandeln.

In dieser Ausführungsform sind die Operationsleuchte 1, die Steuereinrichtung 2, die Kamera 10 und der Monitor 3 mit einem RS 232-Bus verbunden. In einer alternativen Ausführungsform können die einzelnen Baugruppen auch mit separaten Datenleitungen verbunden sein.

Im Betrieb übermittelt die Kamera 10 Videosignale, bei einem YUV-Signal die Helligkeit (Y) und den Farbort (UV-Koordinaten) eines Bildpunkts über die Videoleitung 11 an den Monitor 3. Die empfangenen Bildsignale für den Farbort werden entsprechend der abgespeicherten Korrekturwerte für die eingestellten Farbtemperatur, die basierend auf den über die Signalleitung 12 übermittelten Daten bezüglich der eingestellten Farbtemperatur ausgewählt werden, durch die Steuerung des Monitors 3 verändert.

Die eingestellte Farbtemperatur wird nur bei einer Veränderung über den RS232-Bus über ein spezielles Protokoll an den Monitor 3 übermittelt. Dadurch wird die Datenmenge, die durch den Bus übertragen wird, reduziert, alternativ kann die Übertragung auch zyklisch erfolgen, was die Sicherheit gegen Störungen vergröβert.

Die Signalübertragung zwischen der Steuereinrichtung 2 und dem Monitor 3 erfolgt hier über eine Signalleitung 12, kann aber auch über eine andere Form der Datenübertragung, z.B. eine Infrarot-Schnittstelle oder eine Funkverbindung erfolgen.

Die Helligkeit des Monitors 3 wird an einem nicht gezeigten Helligkeitsregler eingestellt. Eine weitere Möglichkeit der Helligkeitseinstellung ist die Kopplung der Monitorhelligkeit an die Helligkeitseinstellung der Operationsleuchte 1. Hierbei gibt die Steuereinrichtung 2 über die Signalleitung 12 ein Signal, das der eingestellten Helligkeit entspricht an den Monitor 3. Der Monitor 3 verändert dementsprechend die Helligkeit des anzeigten Bilds. Je heller die Operationsleuchte 1 leuchtet, um so heller wird auch das Bild des Monitors 3 eingestellt. Diese Kopplung ist abschaltbar, um ungewünschte Helligkeitsveränderungen des Monitors 3 zu vermeiden.

Als eine weitere Möglichkeit ist nur die Unterscheidung der Einstellung in einem Endo-Modus der Operationsleuchte 1 möglich. Im Endo-Modus wird die Operationsleuchte 1 nur mit ca. 10% ihrer maximalen Leuchtstärke betrieben, um dem Operateur eine bessere Erkennbarkeit des am Monitor 3 angezeigten Bilds zu ermöglichen. In dieser Einstellung der Steuerung wird ein entsprechendes Signal von der Steuereinrichtung 2 über die Signalleitung 12 zum Monitor 3 gegeben und der Monitor 3 wird mit einer verminderten Helligkeit betrieben, um eine Blendung des Operateurs zu verhindern. Bei einer Rückkehr aus dem Endo-Modus in eine höhere Beleuchtungsstärke wird das entsprechende Signal von der Steuereinrichtung 2 an den Monitor 3 gegeben und das Bild des Monitors 3 wird auch wieder heller eingestellt.

Eine weitere Funktion ist das automatische Einschalten des Monitors 3 beim Anschließen der Kamera 10. Die Kamera 10 gibt über die Versorgungs- und Signalleitung 13 an die Steuereinrichtung 2 ein entsprechendes Signal, sobald sie über die Steckverbindung verbunden wird. Dadurch erkennt die Steuerungsvorrichtung 2, dass die Kamera 10 an die Operationsleuchte 1 angeschlossen wird, und gibt über die Signalleitung 12 ein entsprechendes Signal an den Monitor 3 weiter. Es erfolgt dann eine automatische Aktivierung des Monitors 3 aus dem Standby-Modus, sowie eine Umschaltung auf einen entsprechenden Eingang mit anliegendem Signal.

Alternativ zur Übermittlung der Information, mit welcher Farbtemperatur die Operationsleuchte 1 betrieben wird, kann auch ein Sensor zur Erfassung der Farbtemperatur des ausgestrahlten Lichts vorhanden sein. Die Sensordaten werden dann an die Steuereinrichtung 2 weitergegeben, entsprechend aufbereitet und über die Signalleitung 12 an den Monitor 3 gegeben, der damit die Farbtemperaturkorrektur durchführt. Optional kann auch der Farbtemperatursensor ein entsprechend aufbereitetes Signal an den Monitor 3 schicken, der damit die Farbtemperaturkorrektur durchführt.

Die Kamera 10 muss nicht zwangsweise an der Operationsleuchte 1 befestigt sein. Eine Anbringung am Tragsystem der Operationsleuchte 1 oder auf einem separaten Stativ ist ebenfalls möglich.

Die Verwendung einer beliebigen Kamera 10 ist möglich, da der Weißabgleich der Kamera 10 fest eingestellt bleiben kann und die Veränderung der Farbtemperatur des beleuchteten Operationsfelds direkt von der Operationsleuchte 1 an den Monitor oder das Bildverarbeitungsgerät 3 gegeben wird.

## Patentansprüche

1. System mit einer Operationsleuchte (1), deren Farbtemperatur mit Hilfe einer Steuereinrichtung (2) einstellbar ist, einer Kamera (10) und einem Monitor (3), **dadurch gekennzeichnet, dass** Mittel (12) zur Übertragung der eingestellten, oder von einem Sensor erfassten und an die Steuereinrichtung (2) weitergegebenen Farbtemperatur an den Monitor (3) vorgesehen sind.

2. System gemäß Anspruch 1, **dadurch gekennzeichnet, dass** der Monitor (3) mit der Steuereinrichtung (2) der Operationsleuchte (1) über eine Schnittstelle und eine Signalübertragungsstrecke (12) verbunden ist.

3. System gemäß Anspruch 1 oder 2, **dadurch gekennzeichnet, dass** der Monitor (3) mit dem Signalausgang der Kamera (10) verbunden ist.

4. System gemäß einem der Ansprüche 1 bis 3, **dadurch gekennzeichnet, dass** ein Sensor oder eine Baugruppe zur Erfassung der Farbtemperatur vorgesehen ist.

5. System gemäß einem der vorhergehenden Ansprüche, **dadurch gekennzeichnet, dass** der Monitor (3) Mittel zur Speicherung der Korrekturwerte in Abhängigkeit von der Farbtemperatur aufweist.

6. System gemäß einem der vorangehenden Ansprüche, **dadurch gekennzeichnet, dass** der Monitor (3) an einem Tragarm des Operationsleuchtensystems angeordnet ist.

7. System gemäß einem der Ansprüche 1 bis 6, **dadurch gekennzeichnet, dass** die Übertragung oder Erfassung der Farbtemperatureinstellung zyklisch erfolgt.

8. System gemäß einem der Ansprüche 1 bis 6, **dadurch gekennzeichnet, dass** die Übertragung oder Erfassung der Farbtemperatureinstellung bei Änderung erfolgt.

9. System gemäß einem der vorhergehenden Ansprüche, **dadurch gekennzeichnet, dass** die automatische Aktivierung des Monitors (3) aus dem Standby-Modus bei Erkennung der Kamera (10) vom Leuchtensystem erfolgt.

10. System gemäß einem der vorhergehenden Ansprüche, **dadurch gekennzeichnet, dass** Mittel zur Übertragung der eingestellten Helligkeit an den Monitor (3) vorgesehen sind

11. System gemäß einem der Ansprüche 2 bis 10, **dadurch gekennzeichnet, dass** die Signalübertragungsstrecke (12) ein Signalkabel ist.

12. System gemäß einem der Ansprüche 2 bis 10, **dadurch gekennzeichnet, dass** die Signalübertragungsstrecke (12) eine Infrarotstrecke ist.

13. System gemäß einem der Ansprüche 2 bis 10, **dadurch gekennzeichnet, dass** die Signalübertragungsstrecke (12) eine Funkstrecke ist.

14. System gemäß einem der vorangehenden Ansprüche, **dadurch gekennzeichnet, dass** die Kamera (10) mit der Steuereinrichtung (2) verbunden ist.

15. System gemäß einem der vorangehenden Ansprüche, **dadurch gekennzeichnet, dass** die Steuereinrichtung (2), die Kamera (10) und der Monitor (3) über einen Bus verbunden sind.

## Claims

1. System with
a surgical lamp (1), the color temperature thereof is adjustable by means of a control device (2),
a camera (10) and
a monitor (3), **characterized in that**
means (12) for transmitting the color temperature which is set or detected by a sensor and transmitted to the control device (2) to the monitor (3) are provided.

2. System according to claim 1, **characterized in that**
the monitor (3) is connected to the control device (2) of the surgical lamp (1) via an interface and a signal transmission link (12).

3. System according to anyone of claims 1 and 2, **characterized in that**
the monitor (3) is connected to the signal output of the camera (10).

4. System according to anyone of claims 1 to 3, **characterized in that**
a sensor or an assembly for detecting the color temperature are provided.

5. System according to anyone of the preceding claims, **characterized in that**
the monitor (3) comprises means for storing the correction values in accordance to the color temperature.

6. System according to anyone of the preceding claims, **characterized in that**
the monitor (3) is arranged at a boom of the surgical lamp system.

7. System according to anyone of claims 1 to 6, **characterized in that**
the transmission or detection of the setting of the color temperature is periodically performed.

8. System according to anyone of claims 1 to 6, **characterized in that**
the transmission or detection of the setting of the color temperature is performed when changed.

9. System according to anyone of the preceding claims, **characterized in that**
the automatical activation of the monitor (3) from the standby mode is performed when the camera (10) is recognized by the lamp system.

10. System according to anyone of the preceding claims, **characterized in that**
means for transmitting the set brightness to the monitor (3) are provided.

11. System according to anyone of claims 2 to 10, **characterized in that**
the signal transmission link (12) is a signal cable.

12. System according to anyone of claims 2 to 10, **characterized in that**
the signal transmission link (12) is an infrared link.

13. System according to anyone of claims 2 to 10, **characterized in that**
the signal transmission link (12) is a radio communication link.

14. System according to anyone of the preceding claims, **characterized in that**
the camera (10) is connected to the control device (2).

15. System according to anyone of the preceding claims, **characterized in that**
the control device (2), the camera (10) and the monitor (3) are connected via a bus.

## Revendications

1. Système doté d'un éclairage d'opération (1), dont la température de couleur est réglable à l'aide d'un dispositif de commande (2), d'une caméra (10) et d'un moniteur (3), **caractérisé en ce que** des moyens (12) sont prévus pour transmettre au moniteur (3) la température de couleur réglée, ou appréhendée par un capteur et retransmise au dispositif de commande (2).

2. Système selon la revendication 1, **caractérisé en ce que** le moniteur (3) est relié au dispositif de commande (2) de l'éclairage d'opération (1), par l'intermédiaire d'une interface et d'une ligne de transmission de signaux (12).

3. Système selon la revendication 1 ou 2, **caractérisé en ce que** le moniteur (3) est relié à la sortie de signal de la caméra (10).

4. Système selon l'une des revendications 1 à 3, **caractérisé en ce qu'**un capteur ou un composant est prévu pour appréhender la température de couleur.

5. Système selon l'une des revendications précédentes, **caractérisé en ce que** le moniteur (3) comprend des moyens pour mémoriser les valeurs de correction en fonction de la température de couleur.

6. Système selon l'une des revendications précédentes, **caractérisé en ce que** le moniteur (3) est disposé sur un bras support du système d'éclairage d'opération.

7. Système selon l'une des revendications 1 à 6, **caractérisé en ce que** la transmission ou la détection du réglage de température de couleur s'effectue de façon cyclique.

8. Système selon l'une des revendications 1 à 6, **caractérisé en ce que** la transmission ou la détection du réglage de température de couleur s'effectue à l'occurrence d'une modification.

9. Système selon l'une des revendications précédentes, **caractérisé en ce que** l'activation automatique du moniteur (3), à partir du mode d'attente, s'effectue en cas d'identification de la caméra (10) par le système d'éclairage.

10. Système selon l'une des revendications précédentes, **caractérisé en ce que** des moyens sont prévus pour transmettre la luminosité réglée au moniteur (3).

11. Dispositif selon l'une quelconque des revendications 2 à 10, **caractérisé en ce que** la ligne de transmission de signaux (12) est un câble de signalisation.

12. Système selon l'une des revendications 2 à 10, **caractérisé en ce que** la ligne de transmission de signaux (12) est une liaison par infrarouges.

13. Système selon l'une des revendications 2 à 10, **caractérisé en ce que** la ligne de transmission de signaux (12) est une liaison radioélectrique.

14. Système selon l'une des revendications précédentes, **caractérisé en ce que** la caméra (10) est reliée au dispositif de commande (2).

15. Système selon l'une des revendications précédentes, **caractérisé en ce que** le dispositif de commande (2), la caméra (10) et le moniteur (3) sont reliés par un bus.
